# EUROPEAN PATENT APPLICATION

(11) **EP 2 835 421 A1**
(43) Date of publication of application: **11.02.2015**
(21) Application number: 13760804.8
(22) Date of filing: 12.03.2013
(51) Int. Cl.: C12N 1/00, C12N 11/00, C12N 15/09, C12Q 1/04, C12Q 1/68, C12M 1/42

(54) **METHOD FOR PREPARING SOIL MICROORGANISM AND UTILIZATION OF SAME**

(30) Priority: 13.03.2012 JP 2012055306
(71) Applicant: Japan Agency for Marine-Earth Science and Technology, Yokosuka-shi, Kanagawa 237-0061 (JP)
(72) Inventor: KOYAMA, Sumihiro, Yokosuka-shi Kanagawa 237-0061 (JP); TSUBOUCHI, Taishi, Yokosuka-shi Kanagawa 237-0061 (JP)
(74) Representative: Vossius & Partner
(86) International application number: PCT/JP2013/056815
(87) International publication number: WO 2013/137255

(57) **Abstract**

An object of the present invention is to prepare individual microorganisms or a microflora composition derived from land or seafloor soil in which contamination of land or seafloor soil-derived substances is reduced. The present invention provides [1] a method for preparing, from a land or a seafloor soil samples, microorganisms containing a reduced level of land or seafloor soil-derived substances that inhibit nucleic acid analysis, which comprises (1) the step of disposing a suspension of a land or seafloor soil sample containing microorganisms and land or seafloor soil-derived substance that inhibit nucleic acid analysis as an electrolyte on a surface of a substrate at least a part of which constitutes an electrode, and applying a constant potential to the electrode to attach at least a part of the microorganisms to the surface of the substrate, and (2) the step of applying a high-frequency wave potential to the electrode to detach the microorganisms attached to the surface of the substrate in a viable state, and wherein the constant potential applied in the step (1) is greater than -0.5 V but not greater than-0.2 V (vs. Ag/AgCl), or greater than +0.2 V but not greater than +0.4 V (vs. Ag/AgCl), and the high-frequency wave potential applied in the step (2) has a frequency in the range of from 1 kHz to 20 MHz, and a potential range of ±0.1 V (vs. Ag/AgCl) or smaller.

## Description

### Cross reference of related application

This application claims Convention priority based on Japanese Patent Application No. 2012-55306 filed on March 13, 2012 at the Japanese Patent Office. The entire disclosures of Japanese Patent Application No. 2012-55306 are incorporated into the disclosure of the present application.

### Technical Field

The present invention relates to a method for preparing soil microorganisms utilizing constant potential application. The method of the present invention is useful for obtaining microorganisms from a microflora of soil of land or seafloor, which microorganisms contain a reduced level of substances derived from the soil that inhibit nucleic acid analysis, and it is useful for preparation of nucleic acids from soil microorganisms, phylogenetic analysis of soil microorganisms, and the like.

### Background Art

A variety of microorganisms exist in such environments as soil, river, and sea. In recent years, meta-genomic analysis is performed, in which all the nucleic acids of microorganisms contained in an environment are extracted and collected, and nucleotide sequences thereof are comprehensively investigated, and which enables phylogenetic analysis of the microflora in the environment, and prediction of types of metabolisms occurring in the whole environment. The meta-genomic analysis also serves as an important means for discovering genes of useful substances contained in microorganisms viable in a specific environment.

As a method for collecting DNA derived from soil microorganisms from soil, Patent document 1 proposes a method for collecting DNA characterized by adding an anionic substance to soil and a solution containing the microorganisms. It is known that microorganisms derived from a soil sample may firmly bind with particles, organic substance etc. derived from soil, and they act in an inhibitory manner in nucleic acid analysis (Non-patent document 1).

The inventors of the present invention has investigated a novel means that enables disposition and immobilization of living microorganisms as individual single cells or populations containing a small number of microorganisms on a substrate surface, and a means for detachment (deimmobilization) of microorganisms immobilized by using the above novel means in a viable state (Non-patent document 2).

### Prior art references

### Patent document

Patent document 1: Japanese Patent Unexamined Application (Kokai) No. 7-163353

### Non-patent documents

Non-patent document 1: Roh C, Villatte F, Kim BG, Schmid RD, 2006, Comparative study of methods for extraction and purification of environmental DNA from soil and sludge samples, Appl. Biochem. Biotechnol., 134:97-112
Non-patent Document 2: Koyama Set al., 2011, Electrical control of attachment and detachment of living microorganisms using ITO pattern electrode, Abstracts of the 12th Annual Convention of the Japanese Society for Extremophiles, O-11, 44-45

### Summary of the Invention

### Object to be Achieved by the Invention

So far, when a plurality of microorganisms firmly attach to soil microparticles, it has been extremely difficult to directly analyze the microorganisms at the single cell level, even in the analysis of microorganisms utilizing a cell sorter.

An object of the present invention is to prepare individual microorganisms or a microflora composition derived from soil, which contains a reduced level of soil-derived substances.

### Means for Achieving the Object

The inventors of the present invention found that by applying a weak potential that did not produce any electrochemical reaction to an electrolyte consisting of the buffer described in Non-patent document 2 mentioned above not containing any nutrition source, in which living microorganisms (prokaryotes) are dispersed, the living microorganisms can be attached to a cathode substrate. They further found that by applying a high frequency wave potential to the electrode, the microorganisms (prokaryotes) that had been attached to the electrode substrate by the above method could be quantitatively detached and retrieved in a viable state from the electrode surface with almost no damage. Moreover, they also found that, by such detachment, the soil microorganisms could be separated from substances derived from the soil that inhibit nucleic acid analysis. Furthermore, they also found that when nucleic acids were extracted from a microflora composition obtained as described above and used to perform PCR for 16S rRNA analysis, inhibition was not seen, and the analysis could be favorably performed. The present invention was accomplished on the basis of these findings.

The present invention is as set forth below.
[1] A method for preparing, from a land or seafloor soil sample, microorganisms containing a reduced level of land or seafloor soil-derived substances that inhibit nucleic acid analysis, which comprises:
   (1) the step of disposing a suspension of a land or seafloor soil sample containing microorganisms and land or seafloor soil-derived substance that inhibit nucleic acid analysis as an electrolyte on a surface of a substrate at least a part of which constitutes an electrode, and applying a constant potential to the electrode to attach at least a part of the microorganisms to the surface of the substrate, and
   (2) the step of applying a high-frequency wave potential to the electrode to detach the microorganisms attached to the surface of the substrate in a viable state, and wherein:
      the constant potential applied in the step (1) is greater than -0.5 V but not greater than -0.2 V (vs. Ag/AgCl), or greater than +0.2 V but not greater than +0.4 V (vs. Ag/AgCl), and
      the high-frequency wave potential applied in the step (2) has a frequency in the range of from 1 kHz to 20 MHz, and a potential range of ±0.1 V (vs. Ag/AgCl) or smaller.
[2] The preparation method according to [1], wherein the electrolyte used in the step (1) does not contain any source of nutrition for the microorganisms.
[3] The method according to [1] or [2], wherein the electrolyte used in the step (1) has a salt concentration of 10 g/L or lower.
[4] The method according to [1] or [2], wherein the electrolyte used in the step (1) comprises a phosphate buffer (Ca²⁺ and Mg²⁺ free).
[5] The method according to [1], wherein the electrolyte used in the step (1) comprises artificial seawater or natural seawater, and the constant potential applied in the step (1) is greater than -0.4 V but not greater than -0.2 V (vs. Ag/AgCl), or greater than +0.2 V but not greater than +0.4 V (vs. Ag/AgCl).
[6] The method according to any one of [1] to [5], wherein the high-frequency wave potential is a rectangular wave, sine wave, or triangle wave.
[7] The method according to any one of [1] to [6], wherein the entire surface of the substrate constitutes the electrode.
[8] The preparation method according to any one of [1] to [7], wherein, after the step (2) is performed, the steps (1) and (2) are further performed.
[9] The preparation method according to any one of [1] to [8], which is for preparing the microorganisms in the form of a microflora composition.
[10] A method for preparing a nucleic acid, which comprises the steps defined in any one of [1] to [9], and further comprises the step of extracting a nucleic acid from obtained microorganisms or microflora composition.
[11] A method for phylogenetic analysis of a microflora contained in a land or seafloor soil sample, which comprises the steps defined in [9], and further comprises the step of extracting a nucleic acid from an obtained microflora composition, and analyzing the extracted nucleic acid.

### Effect of the Invention

According to the present invention, microorganisms (prokaryotes) can be attached to an electrode surface in a viable state. Further, according to the present invention, microorganisms attached to an electrode surface can be retrieved in a viable state. These methods can be performed for *Escherichia coli,* which is a Gram-negative bacterium, or *Bacillus subtilis,* which is a Gram-positive bacterium.

The present invention enables comparative analysis of gene or protein expression for single cells utilizing a high performance light microscope such as a confocal laser scanning microscope, like the single cell analysis currently performed for animal cells, by attracting and attaching various microorganisms in soil onto an electrode substrate. Further, the microorganisms can also be retrieved after the analysis.

Further, the method of the present invention makes it possible to electrically remove multi-drug resistant bacteria contaminating blood preparations and the like, and to remove microorganisms present in water, such as bath water.

According to the present invention, soil microorganisms (prokaryotes) or soil microflora composition containing a reduced level of substances that inhibit nucleic acid analysis can be prepared by attaching soil microorganisms to an electrode surface, further detaching and retrieving them. The soil microorganisms may be *Escherichia coli,* which is a Gram-negative bacterium, or *Bacillus subtilis,* which is a Gram-positive bacterium.

The soil microorganisms or soil microflora composition of which level of soil-derived substances that inhibit nucleic acid analysis has been reduced by the present invention can be used for nucleic acid analysis.

### Brief Description of the Drawings

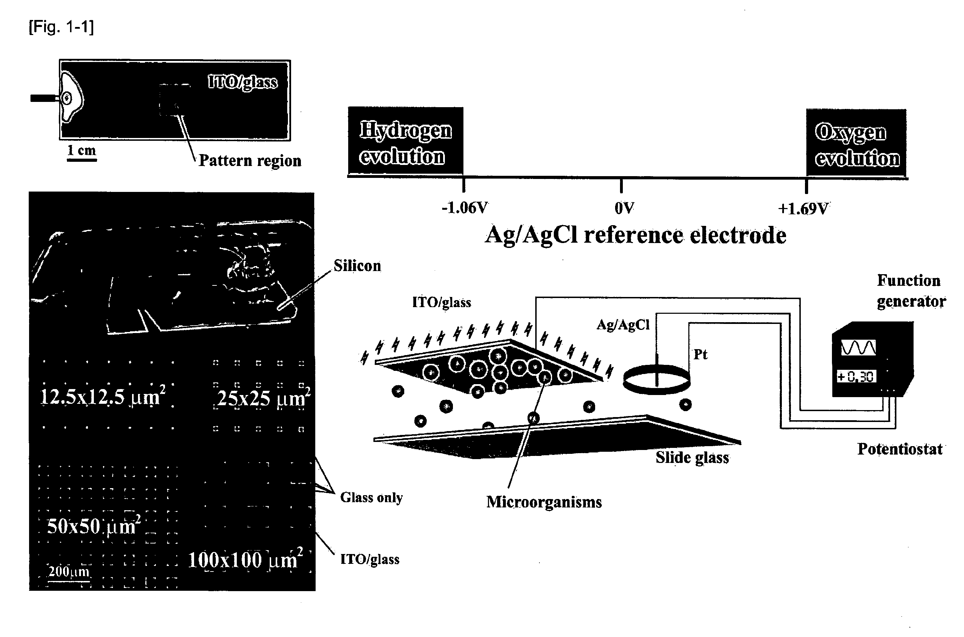

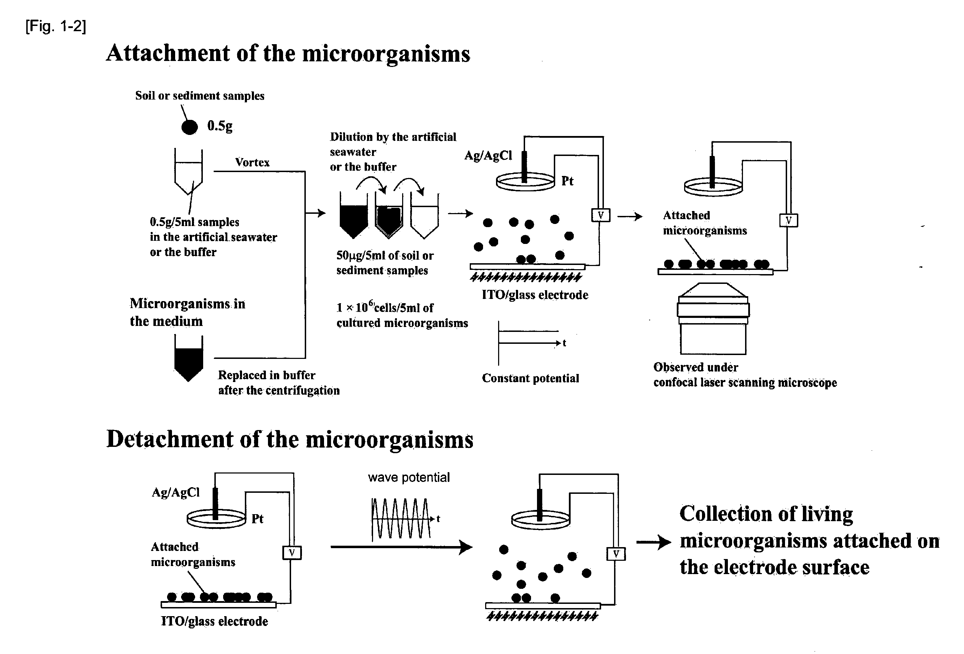

[Figure 1-1] shows a photograph and schematic diagram of electrode chamber.
[Figure 1-2] shows descriptive drawings of attraction and attachment of microorganisms onto electrode and detachment and retrieval of them.
[Figure 2] shows the results of attraction of soil microorganisms onto an electrode performed in Example 1.
[Figure 3] shows the results of attraction and attachment of *Escherichia coli* and *B. subtilis* onto an electrode and detachment and retrieval of them performed in Example 2.
[Figure 4] shows the results of attraction and attachment of soil microorganisms onto an electrode performed in Example 3.
[Figure 5] shows the results of electrical detachment of soil microorganisms performed in Example 4.
[Figure 6] shows the results of attraction and attachment of microorganisms in various buffers performed in Example 5.
[Figure 7] shows the results of attraction and attachment of microorganisms in artificial seawater performed in Example 5.
[Figure 8] shows the results of attraction and attachment of soil microorganisms onto an electrode, as well as detachment and collection of them (Example 6).
[Fig. 9] shows the results of phylogenetic analysis performed in Example 6.
[Fig. 10] shows the results of PCR performed in Example 7.
[Fig. 11] shows the results of phylogenetic analysis performed in Example 8.
[Fig. 12] shows the results of attraction of microorganisms in a medium onto an electrode performed in Comparative Example 1.
[Fig. 13] shows the results of attraction of microorganisms in 280 mM mannitol aqueous solution onto an electrode performed in Comparative Example 2.

### Modes for Carrying out the Invention

### <Method for immobilizing living microorganisms>

The first aspect of the present invention relates to a method for immobilizing living microorganisms.

This method comprises the step (1) of disposing a suspension containing microorganisms as an electrolyte on a surface of a substrate at least a part of which constitutes an electrode, and applying a constant potential to the electrode to attach at least a part of the microorganisms to the surface of the substrate. The method is further characterized in that the constant potential applied in the step (1) is greater than - 0.5 V but not greater than -0.2 V (vs. Ag/AgCl), or greater than +0.2 V but not greater than +0.4 V (vs. Ag/AgCl), and the electrolyte used in the step (1) does not contain any source of nutrition for the microorganisms.

### Step (1)

The step (1) is a step of applying a constant potential on a surface of a substrate at least a part of which constitutes an electrode. The substrate at least a part of which constitutes an electrode is not particularly limited. The entire surface of the substrate can constitute an electrode, or a part thereof can constitute an electrode and another part can be a non-electrode (substrate). The substrate at least a part of which constitutes an electrode may consist of, for example, a substrate that is not an electrode on which an electrode layer is provided, or a substrate of which entire body constitutes an electrode, such as a carbon electrode. When an electrode layer is provided on a substrate that is not an electrode, the electrode can be present on a part or all of the surface of an insulating substrate. A substrate having such an electrode can be obtained by, for example, coating indium tin oxide (ITO) on a glass slide. However, the insulating substrate is not limited to a glass slide, and is not particularly limited so long as it is a non-electrically conductive solid. It can be comprised of a non-electrically conductive organic or inorganic material. Examples of non-electrically conductive organic and inorganic materials include, in addition to glass, plastics and ceramics. The electrode is not limited to indium tin oxide (ITO), and an electrode of any known electrode material can be appropriately employed.

When an electrode layer is provided on a substrate, the electrode layer can be provided over the entire surface of the substrate, or the electrode layer can be provided over a part of the surface of the electrode substrate. When an electrode layer is provided over a part of a surface of an electrode substrate, the electrode layer can be provided, for example, in the form of an array or stripes. The size (area or dimension) of the electrode layer can be appropriately determined. For example, the area of the electrode can fall within the range of 1 to 900 cm².

The term "in the form of an array" means, for example, that the electrode layer is arranged as multiple minute regions disposed in columns or rows. The number of minute regions in the columns or rows is not particularly limited, and can appropriately determined depending on the type (size) of the microorganism, objective use of the substrate on which the microorganism is disposed in an array, and the like. For example, the regions may be arranged in a number in the range of 10 to 10⁵ vertically by 10 to 10⁵ horizontally. However, this range is not a limitation. The shape of the conductive region of the surface of the electrode layer in the form of an array can be rectangular (triangular, square, rectangular, polyhedral, and the like), round, elliptical, or the like, and can be appropriately determined. The dimensions of the conductive regions of the electrode surface in the form of an array can be such that a single microorganism can attach to a single conductive region of the electrode surface. Since microorganisms come in various dimensions, the dimensions of the conductive regions of the electrode surface can be appropriately determined on the basis of the dimensions of the microorganisms to be attached. Further, the dimension of the conductive region of the electrode surface in the form of an array can be such that two or more microorganisms can attach to one conductive region of the electrode surface. Further, the spacing of the individual conductive regions of the electrode can fall within the range of 25 to 100 µm, for example.

In a striped electrode layer, multiple electrode layers in the form of stripes of equal width can be disposed at uniform or varying spacings, or multiple electrode layers in the form of stripes of various widths can be disposed at uniform or varying spacings. The width of the striped electrode layers and the spacing between the striped electrode layers are not particularly limited, and can each independently fall within the range of 25 to 100 µm.

The electrode layer in the form of an array or stripes can be formed on the substrate surface by, for example, coating an electrode layer on the surface of the substrate, forming a mask for an electrode surface in the form of an array or stripes on the surface of the electrode layer, etching the surface of the electrode layer through the mask, and removing the mask. Alternatively, the electrode surface in the form of an array or stripes can be formed on the surface of the substrate by coating the electrode layer on the surface of the substrate through a mask for an electrode surface in the form of an array or stripes, and removing the mask. The formation of the electrode layer, etching of the surface of the electrode layer, and the like can be suitably implemented by the usual methods.

In the step (1), a suspension containing microorganisms is disposed as an electrolyte, a constant potential is applied to the electrode to attach at least a part of the microorganisms to the surface of the substrate. The constant potential applied in the step (1) is greater than -0.5 V but not greater than -0.2 V (vs. Ag/AgCl), or greater than +0.2 V but not greater than +0.4 V (vs. Ag/AgCl). As is specifically indicated in Examples, when the constant potential that is applied to the electrode to which the microorganisms are attached falls within the above range, the microorganisms in the electrolyte specifically attach to the electrode in a viable state. When the constant potential applied to the electrode falls outside the above range, the microorganisms either do not attach to the electrode, or they attach to the electrode, but not in a viable state. The above constant potential has been denoted for an Ag/AgCl reference electrode, but can also be denoted using a reference electrode other than Ag/AgCl. It should be understood that even though a constant potential denoted using a reference electrode other than Ag/AgCl does not fall within the above range, but the constant potential denoted using an Ag/AgCl reference electrode falls within the above range, the constant potential satisfies the requirement of the present invention. When denoted using Ag/AgCl as a reference electrode, -1.06 V is the hydrogen generation potential, and +1.69 V is the oxygen generation potential.

The application period of the constant potential to the electrode can be appropriately determined by taking into account the type of microorganism, the type of electrolyte, the density of the microorganism in the electrolyte, the potential being applied, and the like. For example, it can fall within the range of 1 to 48 hours.

The electrolyte used in the step (1) does not contain a source of nutrition for the microorganisms. Attachment of the microorganisms to the surface of the substrate by the application of the constant potential only occurs when the electrolyte does not contain a source of nutrition for the microorganisms contained in the electrolyte. Although the reason is unclear, the microorganisms do not attach to the electrode when the electrolyte contains a source of nutrition for the microorganisms contained in the electrolyte. The term "source of nutrition for the microorganisms" means a source of nutrition that is essential for cultivation of the microorganism, and consists of some or all of components of a medium that is normally used to cultivate microorganisms. More specifically, the source of nutrition that is not contained in the electrolyte means, for example, sugars, proteins, amino acids, fatty acids, lipids, nucleic acid, and the like.

The term "does not contain a source of nutrition" does not exclude containing trace amounts of sources of nutrition that essentially do not contribute to the growth of the microorganisms. In the course of preparing the electrolyte containing microorganisms from a sample containing the microorganisms, mixing the original electrolyte solution with the sample without removing the trace quantities of sources of nutrition contained in the sample causes them to be passed on to the electrolyte to which the potential is applied. Such an electrolyte containing trace quantities of sources of nutrition falls within the scope of the electrolyte "that does not contain sources of nutrition" defined in the present invention. Although the sample containing the microorganisms can be mixed with the original solution of the electrolyte to prepare the electrolyte, it is possible to filter or the like the sample containing the microorganisms to remove the trace quantities of sources of nutrition contained in the sample, and then mix the sample with the original solution of the electrolyte to reduce the quantity of sources of nutrition passed on with the sample.

The density of the microorganism in the electrolyte is not particularly limited. For example, when the microorganisms are attached to the surface of the electrode cell by cell, the density of the microorganisms in the electrolyte is desirably relatively low. However, since the quantity of microorganisms to be attached to the electrode varies depending on the constant potential applied and with the application period of the constant potential, the density of the microorganism in the electrolyte is one of a number of variables. The density of the microorganism in the electrolyte can be appropriately determined taking these factors into consideration.

It is desirable for the salt concentration in the electrolyte used in the step (1) to be 10 g/L or lower from the viewpoint of attaching the microorganism to the electrode in a viable state. The salt concentration desirably falls within the range of 0 to 10 g/L. Further, it is desirable for the electrolyte used in the step (1) to be a buffer solution from the viewpoint of attaching the microorganisms to the electrode in a viable state. It is also desirable for the salt concentration in the buffer solution to be 10 g/L or lower. The buffer solution is not particularly limited. For example, a phosphate buffer solution (Ca²⁺ and Mg²⁺ free) is desirable from the viewpoint of attaching the microorganisms to the electrode in a viable state. In addition to a phosphate buffer solution, it is also possible to employ 3-morpholinopropanesulfonic acid (MOPS), N-[Tris(hydroxymethyl)methyl]glycine (tricine), N-(2-hydroxyethyl)piperazine-N'-(2-ethanesulfonic acid) (HEPES), and the like. However, from the viewpoint of increasing the rate of attachment of the microorganism to the electrode, a phosphate buffer solution (Ca²⁺ and Mg²⁺ free) is desirable. The pH of the buffer solution can be appropriately selected on the basis of the type of microorganism. For example, it will fall within the range of 5 to 9 for usual microorganisms.

The electrolyte used in the step (1) can be artificial seawater or natural seawater. In these cases, the constant potential applied in the step (1) is desirably greater than -0.4 V but not greater than -0.2 V (vs. Ag/AgCl), or greater than +0.2 V but not greater than +0.4 V (vs. Ag/AgCl). The salt concentration in the case of artificial seawater or natural seawater is about 40 to 50 g/L. When the salt concentration is high, with a constant potential in the range of -0.5 V to -0.4 V, the survival rate of the microorganisms attached to the electrode tends to decrease.

In the method of the present invention, the microorganism that can be attached to the surface of the electrode in a viable state is not particularly limited. As disclosed in Examples, *E. coli,* which is a Gram-positive bacterium, and *B. subtilis,* which is a Gram-negative bacterium, can be attached to the surface of the electrode in a viable state. Examples of the microorganism that can be attached to the electrode surface in a viable state include, but not limited to, *Escherichia coli, Bacillus subtilis, Bacillus halodurans, Shewanella violacea, Shewanella oneidensis, Shewanella surugensis, Kocuria rosea, Kocuria 4B, Shewanella abyssi, Shewanella kaireitica,* and the like.

The present inventors examined the effects of the application of a constant potential on attachment of microorganisms to the electrode substrate also for *Bacillus subtilis, Bacillus halodurans, Shewanella violacea, Kocuria rosea,* and *Shewanella oneidensis.* As a result, it was confirmed that *S. violacea* was strongly drawn onto an electrode to which a constant potential of -0.3 V vs. Ag/AgCl was applied in artificial seawater, and the other microorganisms were strongly drawn onto electrodes to which a constant potential of -0.4 V vs. Ag/AgCl was applied in a phosphate buffer solution (Ca²⁺ and Mg²⁺ free), like *Escherichia coli.*

### <Method for preparing living microorganisms>

The second aspect of the present invention relates to a method for preparing a live microorganism.

This method comprises:
(1) the step of disposing a suspension containing microorganisms as an electrolyte on a surface of a substrate at least a part of which constitutes an electrode, and applying a constant potential to the electrode to attach at least a part of the microorganisms to the surface of the substrate, and
(2) the step of applying a high-frequency wave potential to the electrode to detach the microorganisms attached to the surface of the substrate in a viable state.

The method is further characterized in that the constant potential applied in the step (1) is greater than -0.5 V but not greater than -0.2 V (vs. Ag/AgCl), or greater than +0.2 V but not greater than +0.4 V (vs. Ag/AgCl), the high-frequency wave potential applied in the step (2) has a frequency falling within the range of 1 KHz to 20 MHz and has a potential range of ±1.0 V (vs. Ag/AgCl) or smaller,
the electrolyte used in the step (1) does not contain any source of nutrition for the microorganisms, and
the electrolyte used in the step (2) has a salt concentration of 40 g/L or lower.

The step (1) of the method of the second aspect is identical to the step (1) of the method of the first aspect.

In the step (2), a high-frequency wave potential is applied to the electrode to which the microorganisms have been attached in a viable state in the step (1) to detach the microorganisms that have been attached to the surface of the substrate in a viable state. The microorganisms that have been attached to the electrode and the microorganisms that have attached to a non-electrode surface in the vicinity of the electrode are detached by the application of a high-frequency wave potential. Specifically, the high-frequency wave potential has a frequency falling within the range of 1 KHz to 20 MHz, desirably within the range of 1 to 15 MHz. The potential has, for example, a range of ±0.1 V (vs. Ag/AgCl) or smaller, such as ±10 mV (vs. Ag/AgCl) or smaller, ±9 mV (vs. Ag/AgCl) or smaller, or ±8 mV (vs. Ag/AgCl) or smaller. The waveform of the high-frequency wave potential can be, for example, that of a rectangular wave, sine wave, or triangle wave.

The electrolyte used in the step (2) may have a salt concentration of 40 g/L or less. Even when the electrolyte used in the step (1) is artificial or natural seawater with a salt concentration exceeding 10 g/L, the potential can be adjusted to attach the microorganisms to the surface of the electrode in a viable state. The electrolytes used in the steps (1) and (2) can be of identical or different compositions. Further, the electrolyte used in the step (2) does not contain a source of nutrition, like the electrolyte used in the step (1).

In the step (2), not just the microorganisms on the surface of the electrode, but the microorganisms on the non-electrode surface in the vicinity of the electrode, as well, can be detached. The distance from the electrode of the microorganisms that can be detached on the non-electrode surface depends on the potential and frequency of the high-frequency wave potential. However, it can be about 100 µm or smaller. At the time of applying a high-frequency wave potential, the potential is directly applied to the microorganisms on the electrode and indirectly applied to the microorganisms on the non-electrode surface. Accordingly, there is relatively less stress due to the application of the potential on the microorganisms on the non-electrode surface, and even when damage is inflicted to the microorganisms by the application of the potential (due to the conditions of the high-frequency wave potential), there will tend to be less, or no, damage to the microorganisms on the non-electrode surface. Such an intense high-frequency wave potential that inflicts damage on the microorganisms when applied may sometimes be used because the microorganisms sometimes attach strongly and cannot be readily detached. In such cases, an electrode shape or disposition, or non-electrode surface that allows preferential detachment of the microorganisms on the non-electrode surface can be selected to actively detach the microorganisms on the non-electrode surface.

In the method of the present invention, as shown in Fig. 1-2, microorganisms are attached to the electrode surface of the substrate, or the electrode and non-electrode surfaces in the step (1). For example, the microorganisms to be attached can be in the form of a soil sample, or culture medium containing microorganisms. Such a sample containing microorganisms is appropriately diluted with an electrolyte to adjust it to a prescribed density, after which a constant potential is applied. As needed, the attachment of the microorganisms to the electrode or non-electrode surface by the application of a constant potential can be observed by confocal microscopy or the like.

In the step (2), a high-frequency wave potential is applied to the electrode of the substrate in which microorganisms are attached to the electrode surface, or the electrode and non-electrode surfaces. The microorganisms that have attached to the electrode surface or the electrode and non-electrode surfaces can be detached in a viable state.

In the method of the present invention, the survival rate of the microorganisms that have attached to the electrode surface or electrode and non-electrode surfaces of the substrate in the step (1) depends on the attachment conditions. By way of example, it falls within the range of 50 to 100%. Further, the survival rate of the microorganisms that are detached and retrieved from the electrode surface or electrode and non-electrode surface in the step (2) depends on the detachment conditions. By way of example, it falls within the range of 50 to 100%.

The method of the present invention can comprise a further step of cultivating the detached microorganisms to proliferate them. The method of cultivating the microorganisms can be appropriately selected on the basis of the type of microorganism.

### <Elimination of soil-derived substances that inhibit nucleic acid analysis>

The third aspect of the present invention relates to a method for preparing microorganisms or a microflora composition, which is derived from soil, but contains a reduced level of soil-derived substances that inhibit nucleic acid analysis.

This method comprises:
(1) the step of disposing a suspension of a soil sample containing microorganisms and soil-derived substances that inhibit nucleic acid analysis as an electrolyte on a surface of a substrate at least a part of which constitutes an electrode, and applying a constant potential to the electrode to attach at least a part of the microorganisms to the surface of the substrate, and
(2) the step of applying a high-frequency wave potential to the electrode to detach the microorganisms attached to the surface of the substrate in a viable state.

The steps (1) and (2) of the method of the third aspect are the same as the steps (1) and (2) of the methods of the first and second aspects.

Soil contains various components. The organic substances existing in soil are first divided into organisms and non-organisms, and the non-organisms are secondly divided into dead bodies of animals and plants, and humus. This humus is also called soil organic matter, and divided into non-humic substances consisting of polysaccharides, proteins, amino acids, lipids, lignin, and the like, and humic substances, which is a mixture of high molecular organic substances. Insoluble matter obtained after the humic substances are subjected to an alkaline treatment is called humin. When soluble matter is subjected to an acid treatment, insoluble matter to be obtained is called humic acid, and soluble matter to be obtained is called fulvic acid.

Two kinds of methods are known for recovery of bacterial cell-derived nucleic acids from soil, i.e., a method of separating a bacterial fraction from soil, and recovering nucleic acids from the collected cells, and a method of directly lysing bacterial cells in soil, and then retrieving nucleic acids. However, in both the methods, there may arise problems that cells and nucleic acids are adsorbed by soil-derived substances (for example, humin, humic acid, fulvic acid, etc.), or soil-derived substances inhibit a reaction among reactions for nucleic acid analysis. However, according to the method of the present invention, such problems can be eliminated or ameliorated.

The "soil" referred to in the present invention include soil of land (including farmland, forest, etc.), bottom sediment of river, lake, sea, deep sea, and hot spring, volcanic mud, and volcanic ash, unless especially indicated. The "microflora composition" referred to in the present invention means a composition considered to contain a plurality of microorganisms, which is prepared from soil so as to reflect the composition of the microflora of the soil as it is, unless especially indicated. When it is described in the present invention that "to reduce (reduced level of)" contamination of soil-derived substances concerning microorganisms or microflora composition, it means that soil-derived substances that inhibit analysis of nucleic acids are removed to such an extent that nucleic acid analysis can be effectively performed, unless especially indicated.

The method of the present invention can also be a method for preparing a nucleic acid comprising the step of extracting the nucleic acid from the obtained microorganisms or microflora composition. The nucleic acid may be DNA or RNA.

Since a nucleic acid extracted from a microflora composition obtained by the present invention contains a reduced level of substances that inhibit nucleic acid analysis, it can be used as a sample for nucleic acid analyses such as PCR. In order to sufficiently reduce the substances that inhibit nucleic acid analysis, the aforementioned step (1) and (2) may be repeated, for example, 2 or 3 times.

### Examples

Hereafter, the present invention will be explained in more detail with reference to examples.

### Example 1

According to the procedure shown below, microorganisms were attached to and detached from ITO electrodes in the 3-electrode chamber system shown in Fig. 1-1, and a live/dead determination was made (see Fig. 1-2).
1) A 0.5 g quantity of soil obtained from a vegetable garden in Yokosuka-shi, Kanagawa-ken, Japan, in which no agricultural chemicals had been employed, was added to Dulbecco's PBS(-) (Wako, Osaka, Japan), the overall volume was adjusted to be 5 mL, and the mixture was stirred for 5 minutes with a vortex.
2) The mixture was diluted 10⁴-fold with Dulbecco's PBS(-) to prepare a 10 µg/mL soil sample.
3) To examine whether dead microorganisms would be drawn to the electrode, 10⁴-fold dilution was also conducted with 0.02% (w/v) sodium azide (Wako, Osaka, Japan) dissolved in Dulbecco's PBS(-) to prepare a 10 µg/mL soil sample. Alternatively, soil samples that had been treated for one hour with 70% EtOH at 60°C were employed.
4) The 5 mL soil sample liquid was added to the 3-electrode chamber system (see Figure 1-1), and a -0.4 V vs. Ag/AgCl potential was applied at room temperature for 24 hours.
5) After applying the potential for 24 hours, the soil sample supernatant was retrieved, and the ITO pattern electrode substrate was washed lightly several times with fresh Dulbecco's PBS(-).
6) A 5 mL volume of fresh Dulbecco's PBS(-) was added to the 3-electrode chamber system, and a ±10 mV vs. Ag/AgCl, 9 MHz triangle wave potential was applied for another one hour at room temperature.
7) Following the application of the potential for one hour, the supernatant of the PBS(-) sample was retrieved, and the ITO pattern electrode substrate was washed lightly several times with fresh Dulbecco's PBS(-).
8) The living microorganisms having dehydrogenase activity that had attached to the ITO electrodes obtained after 5) were fluorescence-stained with Bacstain CTC Rapid Staining Kit for Microscopy (Dojindo, Kumamoto, Japan), and then observed by confocal laser microscopy (FV500, Olympus, Tokyo, Japan).
9) The microorganisms that had attached to the ITO electrodes obtained after 5) and 7) were fluorescence-stained with LIVE/DEAD BacLight Bacterial Viability Kit (Molecular probes, Eugene, OR, USA), and a determination was made as to whether they were alive or dead by confocal laser microscopy (FV500).
10) The microorganisms that had been retrieved in the supernatant samples retrieved in 5) and 7) were fluorescence-stained with LIVE/DEAD BacLight Bacterial Viability Kit, and a determination was made as to whether they were alive or dead by confocal laser microscopy (FV500) using a hemacytometer.

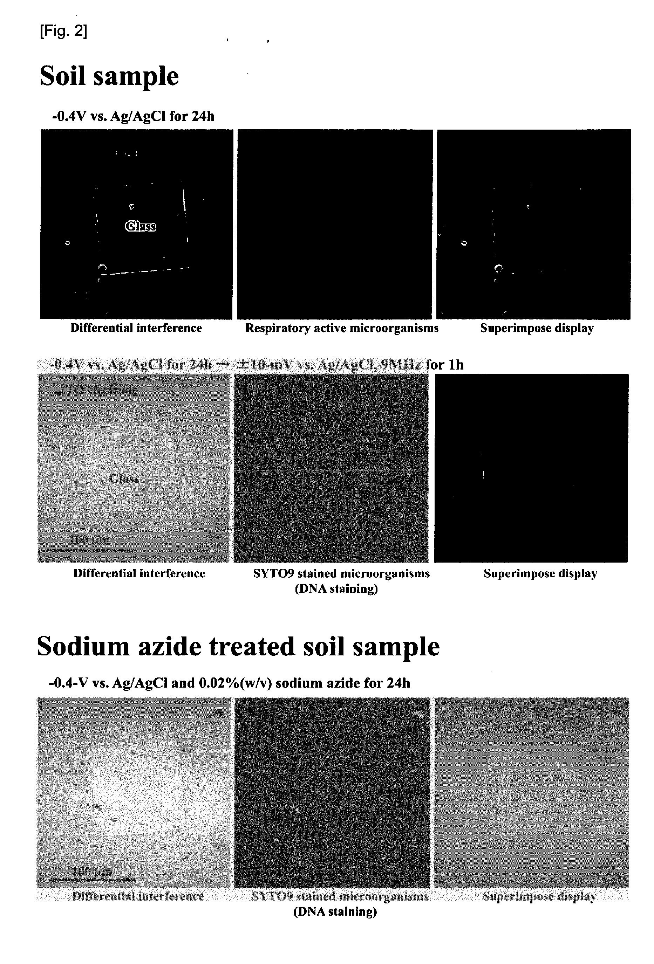

The results are given in Figure 2.

The living microorganisms (prokaryotes) in the soil could be attached to the electrode substrate to which a weak negative potential had been applied without producing any electrochemical reaction (see the upper portion of Figure 2). Dead microorganisms did not attach to the surface of the electrode to which a slightly negative potential was applied (see the lower portion of Figure 2).
(2) The application of a high-frequency wave potential (±10 mV vs. Ag/AgCl, 9 MHz triangle wave) to the microorganisms (prokaryotes) that had attached to the electrode substrate could result in the detachment and retrieval of 99% of the microorganisms from the electrode surface with almost no damage (middle portion of Figure 2). The survival rate of the microorganisms remaining on the electrode surface one hour after the application of the high-frequency wave potential was 91% (= 517/566 cells). The retrieval rate of live bacteria from the soil was 84% (= 154/183). Measurement with a hemacytometer revealed that 3 x 10⁶ microorganisms had been successfully retrieved from 50 micrograms of soil.

### Example 2

(1) According to the procedure shown below, microorganisms were attached to and detached from ITO electrodes in the 3-electrode chamber system shown in Fig. 1-1, and a live/dead determination was made (see Fig. 1-2).
   *1) Escherichia coli* or *Bacillus subtilis* was cultured overnight in 5 mL of the Luria-Bertani medium (LB medium, Difco Laboratories, Inc., Detroit, MI, USA) at 37°C and 120 rpm.
   2) The medium was centrifuged, the supernatant was discarded, and the pellet was resuspended in 5 mL of Dulbecco's PBS(-).
   3) A cell count was taken with a hemacytometer, and the microorganisms were poured into a 3-electrode chamber system at 1 x 10⁶ cells/well.
   4) A -0.4 V vs. Ag/AgCl potential was applied for 24 hours at room temperature.
   5) After applying the potential for 24 hours, the supernatant was retrieved, and the ITO pattern electrode substrate was washed lightly several times with fresh Dulbecco's PBS(-).
   6) A 5 mL volume of fresh Dulbecco's PBS(-) was added to the 3-electrode chamber system, and a ±10 mV vs. Ag/AgCl, 9 MHz triangle wave potential was applied for another one hour at room temperature.
   7) Following the application of the potential for one hour, the supernatant of the PBS(-) sample was retrieved, and the ITO pattern electrode substrate was washed lightly several times with fresh Dulbecco's PBS(-).
   8) The living microorganisms having dehydrogenase activity that had attached to the ITO electrodes obtained after 5) were fluorescence-stained with Bacstain CTC Rapid Staining Kit for Microscopy (Dojindo, Kumamoto, Japan), and then observed by confocal laser microscopy (FV500, Olympus, Tokyo, Japan).
   9) The microorganisms that attached to the ITO electrodes obtained after 5) and 7) were fluorescence-stained with LIVE/DEAD BacLight Bacterial Viability Kit (Molecular probes, Eugene, OR, USA), and a determination was made as to whether they were alive or dead by confocal laser microscopy (FV500).
   10) A 100 µL volume of the supernatant samples retrieved in 5) and 7) were poured onto an LB agar plate, and the colonies were counted.

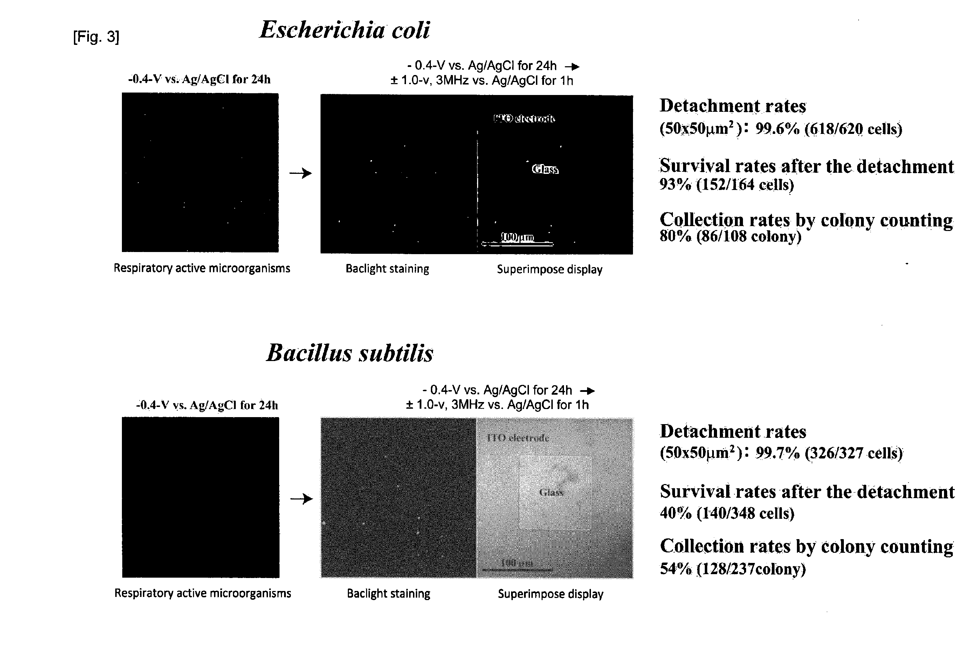

The results are given in Fig. 3. The above electrical attachment was successful for both *Escherichia coli,* a Gram-negative bacterium, and *Bacillus subtilis,* a Gram-positive bacterium. The microorganisms that had been electrically attached were also successfully detached.

The survival rate of the microorganisms remaining on the electrode surface one hour after application of the high-frequency wave potential was 93% (= 152/164 cells) for *Escherichia coli,* and 40% (=140/348 cells) for *Bacillus subtilis.* The retrieval rate of live bacteria from the soil was 80% (= 86/108 colonies) for *Escherichia coli,* and 54% (= 128/237 colonies) for *Bacillus subtilis.*

### Example 3

According to the following procedure, microorganisms in soil were attached to the electrode surface in the same manner as that of Example 1, except that the potential applied was varied.
1) A 0.5 g quantity of soil obtained from a vegetable garden in Yokosuka-shi, Kanagawa-ken, Japan, in which no agricultural chemicals had been employed, was added to Dulbecco's PBS(-) (Wako, Osaka, Japan), the overall volume was adjusted to be 5 mL, and the mixture was stirred for 5 minutes with a vortex.
2) The mixture was diluted 10⁴-fold with Dulbecco's PBS(-) to prepare a 10 µg/mL soil sample.
3) The soil sample liquid in a volume of 5 mL was added to a 3-electrode chamber system, and various constant potentials were applied at room temperature for 24 hours.
4) After applying the potential for 24 hours, the ITO pattern electrode substrate was washed lightly several times with fresh Dulbecco's PBS(-).
5) The living microorganisms having dehydrogenase activity that had attached to the ITO electrodes were fluorescence-stained with a Bacstain CTC Rapid Staining Kit for Microscopy (Dojindo, Kumamoto, Japan), and then observed by confocal laser microscopy (FV500, Olympus, Tokyo, Japan).

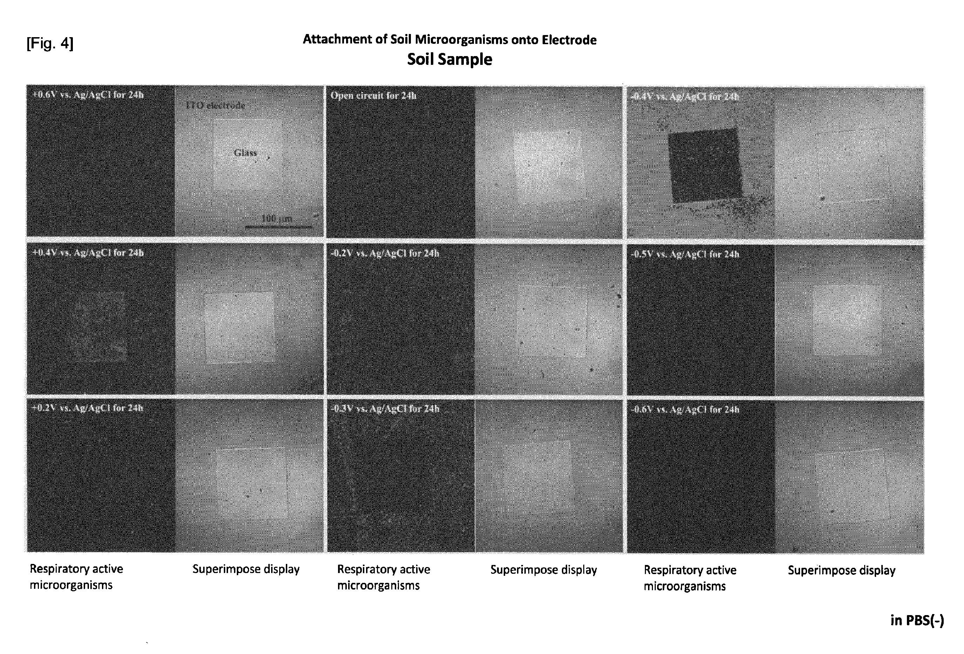

The results are given in Fig. 4. The attachment to the electrode substrate of the microorganisms in soil was confirmed under conditions of the 24 hour application of constant potentials of -0.2 V, -0.3 V, and -0.4 V, vs. Ag/AgCl. Some of the soil microorganisms were confirmed to be of a type that attached even at a constant potential of +0.4 V applied for 24 hours.

### Example 4

According to the following procedure, microorganisms that had been attached to the electrode surface in the same manner as that of Example 1 were detached from the electrode surface by applying various high frequency variation potentials.
1) A 0.5 g quantity of soil obtained from a vegetable garden in Yokosuka-shi, Kanagawa-ken, Japan, in which no agricultural chemicals had been employed, was added to Dulbecco's PBS(-) (Wako, Osaka, Japan), the overall volume was adjusted to be 5 mL, and the mixture was stirred for 5 minutes with a vortex.
2) The mixture was diluted 10⁴-fold with Dulbecco's PBS(-) to prepare a 10 µg/mL soil sample.
3) The soil sample in a volume of 5 mL was added to a 3-electrode chamber system (see Figure 1-1), and a -0.4 V vs. Ag/AgCl potential was applied at room temperature for 24 hours.
4) After applying the potential for 24 hours, the ITO pattern electrode substrate was washed lightly several times with fresh Dulbecco's PBS(-).
5) A 5 mL volume of fresh Dulbecco's PBS(-) was added to the 3-electrode chamber system, and ±4 mV, ±6 mV, ±8 mV, or ±10 mV vs. Ag/AgCl, 9 MHz triangle wave potential was applied for another one hour at room temperature.
6) Following the application of the potential for one hour, the ITO pattern electrode substrate was washed lightly several times with fresh Dulbecco's PBS(-).
7) The living microorganisms having dehydrogenase activity that had attached to the ITO electrodes obtained after 4) were fluorescence-stained with a Bacstain CTC Rapid Staining Kit for Microscopy (Dojindo, Kumamoto, Japan), and then observed by confocal laser microscopy (FV500, Olympus, Tokyo, Japan).
8) The microorganisms that had attached to the ITO electrodes obtained after 6) were fluorescence-stained with LIVE/DEAD BacLight Bacterial Viability Kit (Molecular probes, Eugene, OR, USA), and a determination was made as to whether they were alive or dead by confocal laser microscopy (FV500).
9) The detachment rate was calculated from the quantity of microorganisms per 50 x 50 µm² that had attached to the ITO electrodes obtained after 4) and 6).

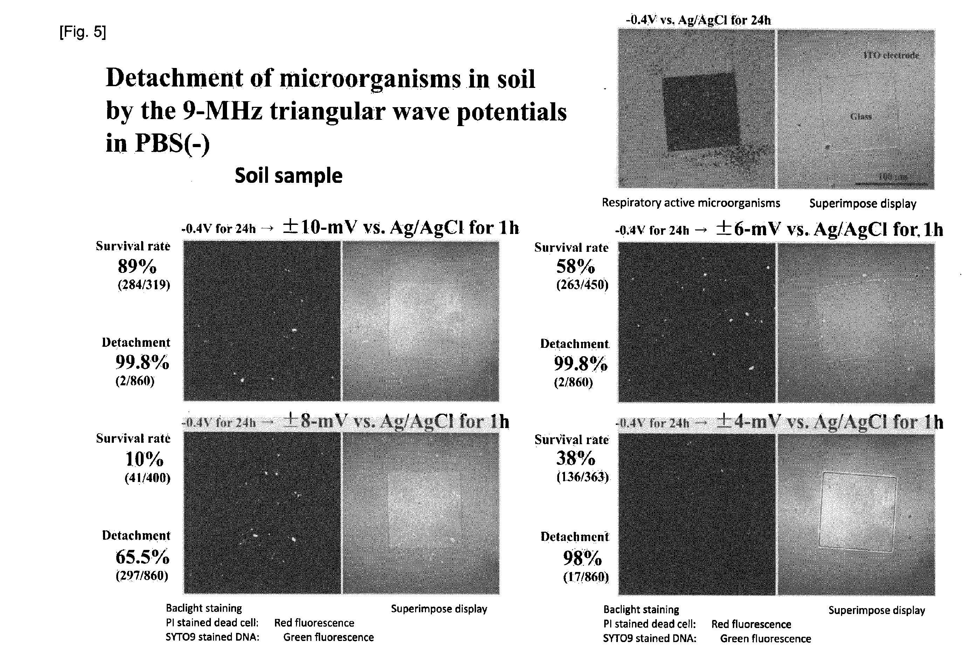

The results are given in Fig. 5. The microorganism survival rates and the detachment rates from the electrode substrate when the various potentials had been applied for one hour are given. Detachment occurred with the best conditions of a survival rate of 89% and a detachment rate of 99.8% at ±10 mV.

### Example 5

According to the following procedure, microorganisms in soil were attached to the electrode surface in the same manner as that of Example 1, except that the electrolyte was variously changed.
1) 10 g/L of MOPS (Dojindo), Tricine (Dojindo), and HEPES (cell culture tested, Sigma, St. Louis, MO, USA) were adjusted to pH 7.
2) Artificial seawater (30 g/L NaCl, 0.7 g/L KCl, 5.3 g MgSO₄-7H₂O, 10.8 g MgCl₂-6H₂O, 1.0 g/L CaSO₄-2H₂O) was prepared.
3) A 0.5 g quantity of soil obtained from a vegetable garden in Yokosuka-shi, Kanagawa-ken, Japan, in which no agricultural chemicals had been employed, was added to each of the various buffers and artificial seawater, the overall quantity was adjusted to 5 mL, and the mixture was stirred for 5 minutes with a vortex.
4) Each mixture was diluted with each of the various buffers and seawater 10⁴-fold to prepare a 10 µg/mL soil sample.
5) The soil sample in a volume of 5 mL was added to a 3-electrode chamber system, and various constant potentials were applied at room temperature for 24 hours.
6) After applying the potential for 24 hours, the ITO pattern electrode substrate was washed lightly several times with fresh Dulbecco's PBS(-).
7) The living microorganisms having dehydrogenase activity that had been attached to the ITO electrodes were fluorescence-stained with Bacstain CTC Rapid Staining Kit for Microscopy (Dojindo, Kumamoto, Japan), and then observed by confocal laser microscopy (FV500, Olympus, Tokyo, Japan).

### (1) Various buffers

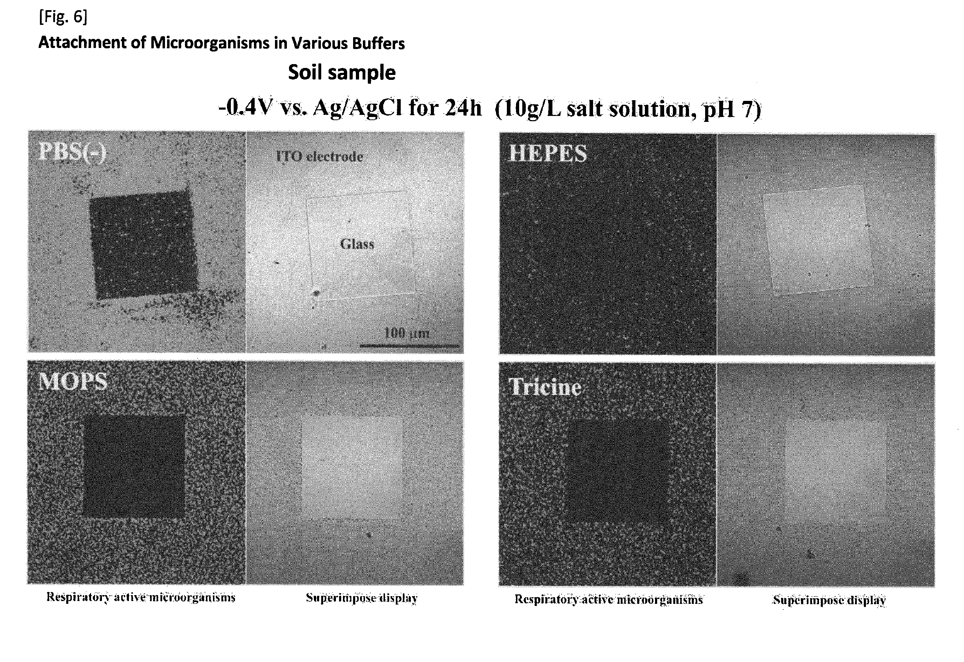

The results are given in Fig. 6. The microorganisms were electrically adsorbed in 10 g/L of various buffers for 24 hours at -0.4 V vs. Ag/AgCl. As a result, PBS(-) exhibited the best electric adsorption, followed by MOPS buffer and Tricine buffer. HEPES somewhat blocked the adsorption of the microorganisms to the electrodes.

### (2) Artificial seawater

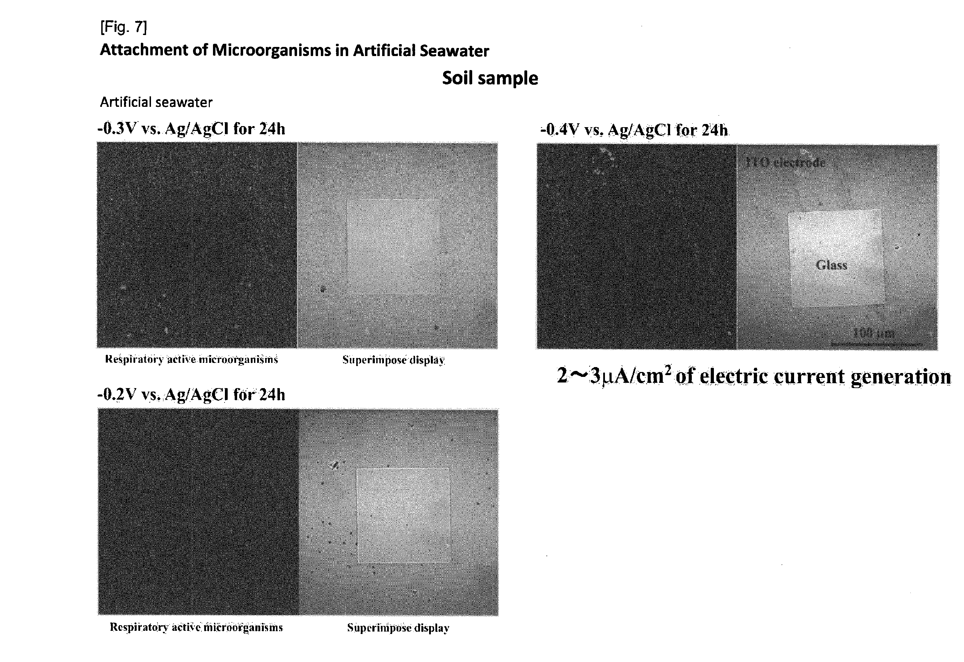

The results are given in Fig. 7. Between -0.3 V and +0.6 V vs. Ag/AgCl, no current flowed in the artificial seawater, and so no damage could be confirmed for the adsorbed microorganisms. The optimal condition for electric adsorption of microorganisms in artificial seawater was -0.3 V vs. Ag/AgCl.

### Example 6: Phylogenetic analysis of bacteria existing in deep sea bottom sediment

### 1. Preparation of bacterial flora

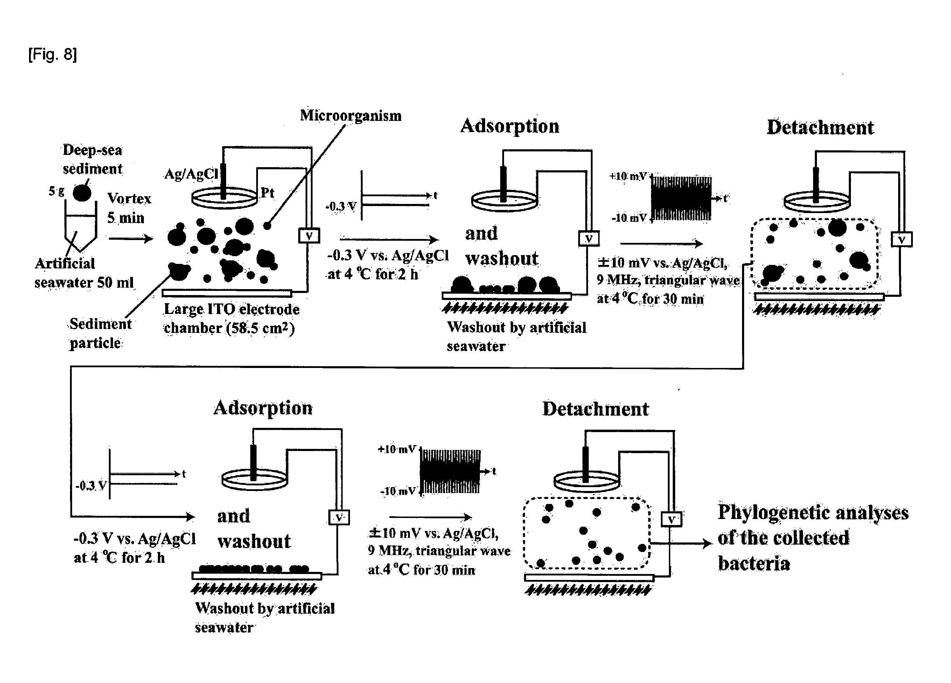

According to the following procedure, microorganisms were twice repeatedly attached to and detached from ITO electrodes of the 3-electrode chamber system shown in Figure 1-1 (see Fig. 8).
1) A 5 g quantity of deep sea bottom sediment collected in an upwelling region of Sagami Bay, Japan at a depth of 1,176 m was added to Dulbecco's PBS(-) (Wako, Osaka, Japan), the overall volume was adjusted to be 5 mL, and the mixture was stirred for 5 minutes with a vortex.
2) The soil sample in a volume of 5 mL was added to a 3-electrode chamber system (see Figure 1-1), and a -0.4 V vs. Ag/AgCl potential was applied at 4°C.
3) After applying the potential for 2 hours, the ITO pattern electrode substrate was washed lightly several times with fresh Dulbecco's PBS(-).
4) A 5 mL volume of fresh Dulbecco's PBS(-) was added to the 3-electrode chamber system, and a ±10 mV vs. Ag/AgCl, 9 MHz triangle wave potential was applied at 4°C.
5) Following the application of the potential for 30 minutes, the ITO pattern electrode substrate was washed lightly several times with fresh Dulbecco's PBS(-).
8) The steps of 2) to 5) were repeated again, and a supernatant PBS(-) sample was collected.

### 2. Phylogenetic Analysis

### 2.1. DNA Extraction

Volume of the electrically detached sample was reduced by lyophilization, and then the sample was subjected to DNA extraction using ISOIL Large for Beads ver. 2 (NIPPON GENE). Further, as a control, DNA was directly extracted from the deep sea bottom sediment subjected to a cell lysis treatment.

### 2.2 Sequence Analysis

### 2.2.1 PCR Amplification

With 20 ng of DNA, PCR was performed by using LA-Taq Polymerase (Takara Bio) with a program of 96°C for 2 min, then 25 cycles of 96°C for 20 sec., 55°C for 15 sec., and 72°C for 90 sec., 72°C for 7 min., and 4°C, ∞. The used primers were the following primers, which are generally used for analysis of bacteria.
Bac 27F: 5'-AGA GTT TGA TCC TGG CTC AG-3' (SEQ ID NO: 1)
Bac 1492R: 5'-GGC TAC CTT GTTACG ACT T-3' (SEQ ID NO: 2)

### 2.2.2 Electrophoresis, extraction and purification

Since the amount of the amplification sample was small, and it was desired to obviate damage by UV, SyBr Green I was used as a staining material. For gel extraction, NucleoSpin Extract II (Takara Bio) was used.

### 2.2.3 Subcloning and transformation

pT7 Blue2 T-vector (Takara Bio), which is a TA cloning vector, was used. As ligase, Ligation High2 (Toyobo Co., Ltd.) was used. The *Escherichia coli* DH5 strain was transformed with the ligation sample, and positive clones were picked up on the LB medium (ampicillin, IPTG, and X-Gal selection medium), and cultured in the LB medium (ampicillin) on a 96-well plate. Colony PCR was performed with M13Rv and M13M4, which are vector primers, to select positive clones.

### 2.2.4 Preparation of sample for sequencing

In order to remove primer dimers and unreacted primers, a positive PCR product confirmed by the colony PCR was treated twice with Exo and SAP (Shrimp Alkaline phosphatase) (both from Usb). Then, the Exo and SAP-treated sample was subjected to sequencing reactions in a conventional manner. As the primers, three kinds of primers, M13Rv, and M13M4, which are vector primer, as well as Bac338F, which is a primer for bacteria, were used.
M13Rv: 5'-TGT GGAATT GTG AGC GG-3' (SEQ ID NO: 3)
M13M4: 5'-GTT TTC CCA GTC ACG AC-3' (SEQ ID NO: 4)
Bac338F: 5'-ACT CCT ACG GGA GGC AGC-3' (SEQ ID NO: 5)

### 2.2.5 Sequence Analysis

On the basis of the results of the analysis performed by using three kinds of the primers, contigs were created for every sample, and analyzed by using Mothur (available on http://www.mothur.org/wiki/Main_Page). Specifically, multifastA files of the sequences obtained above (20 clones each in this study) were created, and analyzed by running Mothur on Terminal.

### 2.3 Results

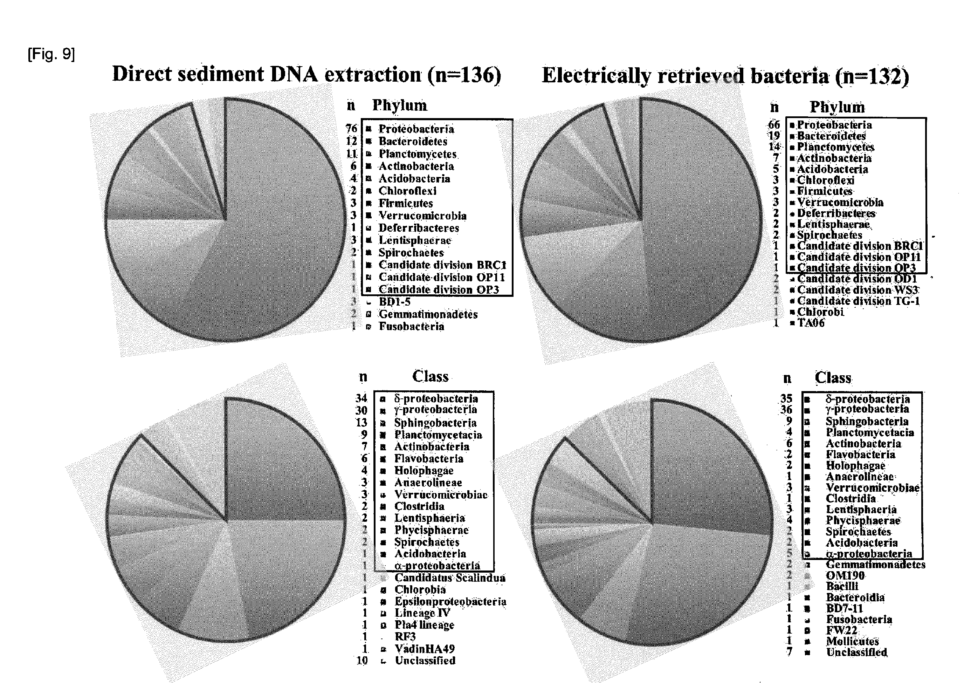

The results are shown in Fig. 9. Almost no difference was observed between the case where DNA directly extracted from deep see sediment was used, and the case where DNA extracted from a microflora composition obtained by performing electric attachment and detachment twice was used.

### Example 7: Phylogenetic analysis of bacteria existing in firm soil

A soil sample was collected from a vineyard in Yokosuka-shi, Kanagawa-ken, Japan, and phylogenetic analysis of bacteria based on 16S rRNA gene was performed. A bacterial flora composition was obtained under the same conditions as those used in Example 6, except that the temperature for the electric attachment and detachment was room temperature. Further, a part of supernatant was collected also after the first attachment and detachment, and subjected to PCR analysis.

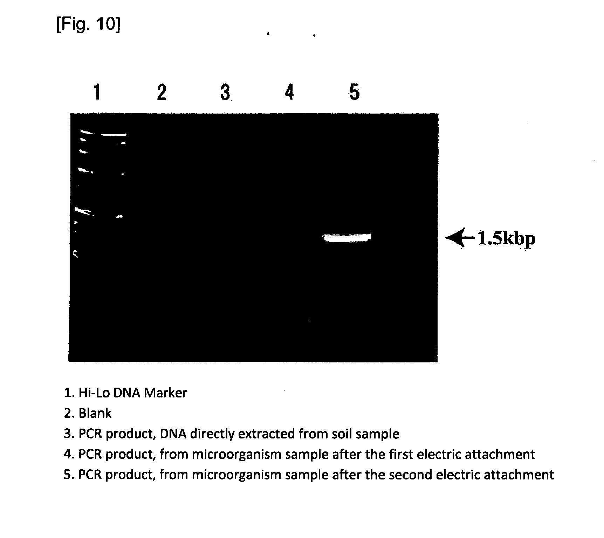

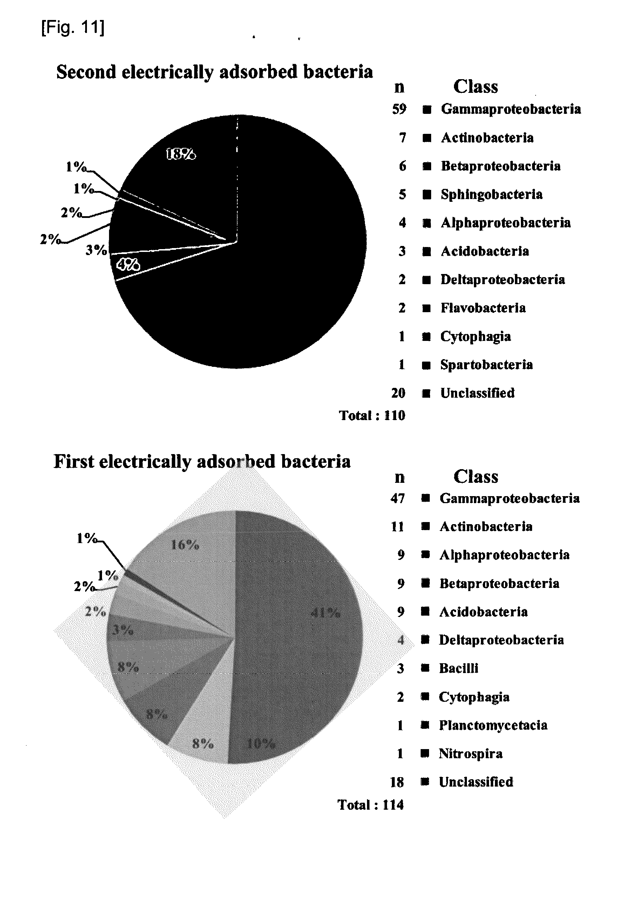

The results are shown in Figs. 10 and 11. When the soil sample was subjected to the electric attachment and detachment twice, substances that inhibit PCR reaction contained in the soil could be sufficiently removed. Almost no difference was observed between the bacteria obtained after the first and second attachment and detachment on the basis of phylogenetic analysis. Further, even though DNAs could be directly collected from the soil sample, PCR for the phylogenetic analysis based on 16S rRNA gene of microorganisms could not be performed with them.

### Comparative Example 1

According to the following procedure, microorganisms in soil were attached to the electrode surface in the same manner as that of Example 1, except that the electrolyte employed was changed to a medium (containing sources of nutrition). Whether soil microorganisms would attach to the electrode substrate even in the medium was examined.
1) A 0.5 g quantity of soil obtained from a vegetable garden in Yokosuka-shi, Kanagawa-ken, Japan, in which no agricultural chemicals had been employed, was added to Dulbecco's PBS(-) (Wako, Osaka, Japan), the overall volume was adjusted to be 5 mL, and the mixture was stirred for 5 minutes with a vortex.
2) The mixture was diluted 10⁴-fold with the Luria-Bertani medium (LB medium, Difco Laboratories, Inc., Detroit, MI, USA) to prepare a 10 µg/mL soil sample.
3) The soil sample in a volume of 5 mL was added to a 3-electrode chamber system, and a -0.4 V vs. Ag/AgCl potential was applied at room temperature for 24 hours.
4) After applying the potential for 24 hours, the ITO pattern electrode substrate was washed lightly several times with fresh Dulbecco's PBS(-).
5) The microorganisms that had attached to the ITO electrodes were fluorescence-stained with LIVE/DEAD BacLight Bacterial Viability Kit (Molecular probes, Eugene, OR, USA), and observed by confocal laser microscopy (FV500).

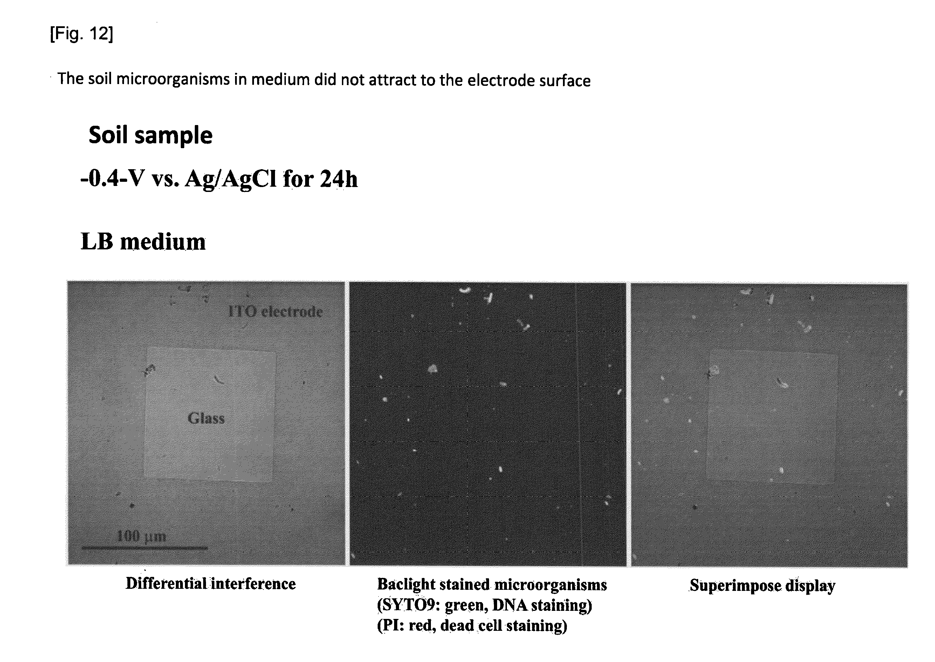

The results are given in Fig. 12. The soil microorganisms were not attracted and did not attach to the electrode substrate in the medium.

### Comparative Example 2-1

According to the following procedure, soil microorganisms were attached to the electrode surface in the same manner as that of Example 1, except that the electrolyte employed was changed to a mannitol solution.
1) A 0.5 g quantity of soil obtained from a vegetable garden in Yokosuka-shi, Kanagawa-ken, Japan, in which no agricultural chemicals had been employed, was added to Dulbecco's PBS(-) (Wako, Osaka, Japan), the overall volume was adjusted to be 5 mL, and the mixture was stirred for 5 minutes with a vortex.
2) The mixture was diluted 10⁴-fold with a 280 mM mannitol aqueous solution to prepare a 10 µg/mL soil sample.
3) The soil sample in a volume of 5 mL was added to a 3-electrode chamber system, and a -0.4 V vs. Ag/AgCl potential was applied at room temperature for 24 hours.
4) After applying the potential for 24 hours, the ITO pattern electrode substrate was washed lightly several times with fresh Dulbecco's PBS(-).
5) The microorganisms that had attached to the ITO electrodes were fluorescence-stained with LIVE/DEAD BacLight Bacterial Viability Kit (Molecular probes, Eugene, OR, USA), and observed by confocal laser microscopy (FV500).

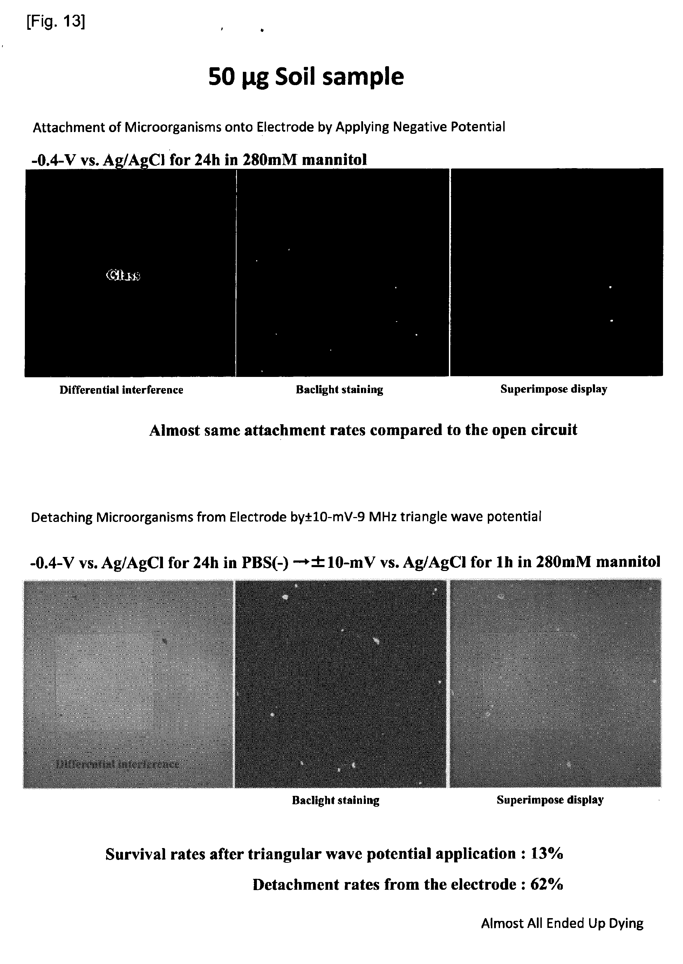

The results are given in the top portion of Fig. 13. In the 280 mM mannitol aqueous solution, the microorganisms attached to the same degree as when no voltage was applied (open circuit) under the conditions of the present invention.

### Comparative Example 2-2

According to the following procedure, soil microorganisms were attached to the electrode surface in the same manner as that of Example 1, except that the electrolyte employed was changed to a mannitol solution, and then detached in a mannitol solution.
1) A 0.5 g quantity of soil obtained from a vegetable garden in Yokosuka-shi, Kanagawa-ken, Japan, in which no agricultural chemicals had been employed, was added to Dulbecco's PBS(-) (Wako, Osaka, Japan), the overall volume was adjusted to be 5 mL, and the mixture was stirred for 5 minutes with a vortex.
2) The mixture was diluted 10⁴-fold with Dulbecco's PBS(-) to prepare a 10 µg/mL soil sample.
3) The soil sample in a volume of 5 mL was added to a 3-electrode chamber system, and a -0.4 V vs. Ag/AgCl potential was applied at room temperature for 24 hours.
4) After applying the potential for 24 hours, the ITO pattern electrode substrate was washed lightly several times with fresh Dulbecco's PBS(-).
5) A 5 mL volume of a 280 mM mannitol aqueous solution was added to the 3-electrode chamber system, and a ±10 mV vs. Ag/AgCl, 9 MHz triangle wave potential was applied for another one hour at room temperature.
6) Following the application of the potential for one hour, the ITO pattern electrode substrate was washed lightly several times with fresh Dulbecco's PBS(-).
7) The microorganisms that had attached to the ITO electrodes were fluorescence-stained with LIVE/DEAD BacLight Bacterial Viability Kit (Molecular probes, Eugene, OR, USA), and observed by confocal laser microscopy (FV500).

The results are given in the lower portion of Fig. 13. Almost none of the attached microorganisms detached under the condition of the application of the high-frequency potential used in the present invention, and almost all ended up dying.

### Industrial Applicability

The present invention is useful in the fields of handling microorganisms.

### Sequence Listing Free Text

SEQ ID NO: 1: PCR primer Bac 27F
SEQ ID NO: 2: PCR primer Bac 1492R
SEQ ID NO: 3: PCR-primer M13Rv
SEQ ID NO: 4: PCR primer M13M4
SEQ ID NO: 5: PCR primer Bac338F

## Claims

1. A method for preparing, from a land or seafloor soil sample, microorganisms containing a reduced level of land or seafloor soil-derived substances that inhibit nucleic acid analysis, which comprises:
(1) the step of disposing a suspension of a land or seafloor soil sample containing microorganisms and land or seafloor soil-derived substance that inhibit nucleic acid analysis as an electrolyte on a surface of a substrate at least a part of which constitutes an electrode, and applying a constant potential to the electrode to attach at least a part of the microorganisms to the surface of the substrate, and
(2) the step of applying a high-frequency wave potential to the electrode to detach the microorganisms attached to the surface of the substrate in a viable state, and wherein:
the constant potential applied in the step (1) is greater than -0.5 V but not greater than -0.2 V (vs. Ag/AgCl), or greater than +0.2 V but not greater than +0.4 V (vs. Ag/AgCl), and
the high-frequency wave potential applied in the step (2) has a frequency in the range of from 1 kHz to 20 MHz, and a potential range of ±0.1 V (vs. Ag/AgCl) or smaller.

2. The preparation method according to claim 1, wherein the electrolyte used in the step (1) does not contain any source of nutrition for the microorganisms.

3. The method according to claim 1 or 2, wherein the electrolyte used in the step (1) has a salt concentration of 10 g/L or lower.

4. The method according to claim 1 or 2, wherein the electrolyte used in the step (1) comprises a phosphate buffer (Ca²⁺ and Mg²⁺ free).

5. The method according to claim 1, wherein the electrolyte used in the step (1) comprises artificial seawater or natural seawater, and the constant potential applied in the step (1) is greater than -0.4 V but not greater than -0.2 V (vs. Ag/AgCl), or greater than +0.2 V but not greater than +0.4 V (vs. Ag/AgCl).

6. The method according to any one of claims 1 to 5, wherein the high-frequency wave potential is a rectangular wave, sine wave, or triangle wave.

7. The method according to any one of claims 1 to 6, wherein the entire surface of the substrate constitutes the electrode.

8. The preparation method according to any one of claims 1 to 7, wherein, after the step (2) is performed, the steps (1) and (2) are further performed.

9. The preparation method according to any one of claims 1 to 8, which is for preparing the microorganisms in the form of a microflora composition.

10. A method for preparing a nucleic acid, which comprises the steps defined in any one of claims 1 to 9, and further comprises the step of extracting a nucleic acid from obtained microorganisms or microflora composition.

11. A method for phylogenetic analysis of a microflora contained in a land or seafloor soil sample, which comprises the steps defined in claim 9, and further comprises the step of extracting a nucleic acid from an obtained microflora composition, and analyzing the extracted nucleic acid.


SEQUENCE LISTING

<110>  Japan Agency for Marine-Earth Science and Technology

<120>  Method for Preparing Soil Microorganisms and Use Thereof

<130>  X3192 EP

<140>  EP 13 76 0804.8
<141>  2013-03-12

<150>  JP 2012-055306
<151>  2012-03-13

<160>  5

<170>  PatentIn version 3.5

<210>  1
<211>  20
<212>  DNA
<213>  Artificial Sequence

<220>
<223>  PCR primer Bac 27F

<400>  1
agagtttgat cctggctcag                                                   20


<210>  2
<211>  19
<212>  DNA
<213>  Artificial Sequence

<220>
<223>  PCR primer Bac 1492R

<400>  2
ggctaccttg ttacgactt                                                    19


<210>  3
<211>  17
<212>  DNA
<213>  Artificial Sequence

<220>
<223>  PCR primer M13Rv

<400>  3
tgtggaattg tgagcgg                                                      17


<210>  4
<211>  17
<212>  DNA
<213>  Artificial Sequence

<220>
<223>  PCR primer M13M4

<400>  4

gttttcccag tcacgac                                                    17

<210>  5
<211>  18
<212>  DNA
<213>  Artificial Sequence

<220>
<223>  PCR primer Bac338F

<400>  5
actcctacgg gaggcagc                                                   18